# EUROPEAN PATENT SPECIFICATION

(11) **EP 4 233 852 B1**
(45) Date of publication and mention of the grant of the patent: **23.10.2024**
(21) Application number: 21881489.5
(22) Date of filing: 26.03.2021
(51) Int. Cl.: A61K 31/135, A61K 47/36, A61P 25/16, A61P 25/28, A61K 9/00, A61K 47/40, A61K 47/38, A61K 47/32

(54) **SUBLINGUAL FILM DOSAGE OF RASAGILINE OR PHARMACEUTICALLY ACCEPTABLE SALT THEREOF, AND PREPARATION METHOD THEREFOR AND USE THEREOF**
SUBLINGUALE FILMDOSIERUNG VON RASAGILIN ODER PHARMAZEUTISCH UNBEDENKLICHEM SALZ DAVON SOWIE HERSTELLUNGSVERFAHREN DAFÜR UND VERWENDUNG DAVON
DOSAGE DE FILM SUBLINGUAL DE RASAGILINE OU D'UN SEL PHARMACEUTIQUEMENT ACCEPTABLE ASSOCIÉ ET SON PROCÉDÉ DE PRÉPARATION ET UTILISATION ASSOCIÉE

(30) Priority: 23.10.2020 CN 202011145109
(43) Date of publication of application: 30.08.2023
(73) Proprietor: SPH Zhongxi Pharmaceutical Co., Ltd., Shanghai 201806 (CN); Shanghai Modern Pharmaceutical Engineering Research Center Co., Ltd., Shanghai 201203 (CN)
(72) Inventor: CHEN, Fang, Shanghai 201203 (CN); DING, Yixin, Shanghai 201806 (CN); WANG, Bing, Shanghai 201203 (CN); LI, Dexiang, Shanghai 201806 (CN); YANG, Liuliu, Shanghai 201203 (CN); FU, Linyong, Shanghai 201806 (CN); ZHOU, Min, Shanghai 201806 (CN); DING, Yunhui, Shanghai 201806 (CN)
(74) Representative: Dehns
(86) International application number: PCT/CN2021/083192
(87) International publication number: WO 2022/083063

(56) References cited:
- CN-A- 103 239 427
- CN-A- 105 616 389
- CN-A- 105 616 389
- RAO PINGALI SRINIVASA ET AL: "Formulation, Optimization and Evaluation of Rasagiline Mouth Dissolving Films", INTERNATIONAL JOURNAL OF PHARMACEUTICAL SCIENCES AND NANOTECHNOLOGY, vol. 11, no. 6, 30 November 2018 (2018-11-30), IN, pages 4323 - 4331, XP093052578, ISSN: 0974-3278, Retrieved from the Internet <URL:https://www.ijpsnonline.com/index.php/ijpsn/article/download/400/315> [retrieved on 20230607], DOI: 10.37285/ijpsn.2018.11.6.5

## Description

The present application claims the priority of Chinese patent application 2020111451096 filed on October 23, 2020.

### TECHNICAL FIELD

The present disclosure relates to a sublingual film of Rasagiline or a pharmaceutically acceptable salt thereof, and a preparation method therefor and a use thereof.

### BACKGROUND

Parkinson's disease (PD), the second most common neurodegenerative disease, mostly occurs in people over the age of 40, and 2-3% of the elderly over the age of 65 are afflicted by this disease. Symptoms are different for each Parkinson's patient, but the most common symptoms are movement disorders and stiffness of skeletal muscles, tremors, slowness of movement, poor balance and gait disturbance. In addition, it is often accompanied by depression, sleep disturbance, dizziness, dementia, speech impairment, respiratory system disease and dysphagia, and the symptoms will worsen year by year, and even lead to death.

Currently, the drugs for treating Parkinson's disease mainly include Levodopa, DA receptor agonists, MAO-B inhibitors, COMT inhibitors, anticholinergic agents, etc., wherein the most common one is Levodopa. Although Levodopa can quickly improve Parkinson's symptoms and has irreplaceable advantages, its advantage is merely in the initial few years of treatment, and the clinical effect will be significantly weakened after long-term use, and there are serious side effects, including symptoms like movement disorders, motor fluctuations (commonly known as the "on-off phenomenons"), and mental confusions, etc.

Rasagiline mesylate is a potent, selective and irreversible MAO-B inhibitor used in the treatment of Parkinson's disease, Alzheimer's disease and a variety of other disorders by inhibiting MAO in the brain. Metabolism of Rasagiline does not produce Amphetamine and does not cause unwanted sympathomimetic effects. Compared with Levodopa, Rasagiline has better safety and can be used alone for the treatment of patients with early Parkinson's disease. Currently, the marketed formulation of Rasagiline mesylate is an ordinary tablet, with the product name AZILECT, which was first launched in Israel in March 2005, in the UK in June, in Ireland in September, in the United States in 2006, and officially launched in China in 2017. The specifications are 0.5 mg and 1 mg (calculated as Rasagiline). The formulation patents on Rasagiline and pharmaceutically acceptable salts thereof are all oral administration formulations, such as Rasagiline orally disintegrating composition (CN101098685A), a Rasagiline formulation and a preparation method therefor (CN103315983A), a Rasagiline tablet (CN105496979A), a Rasagiline tablet and a preparation method therefor (CN107753446A), Rasagiline oral solid formulations (CN1911211A), orally disintegrating tablets of Rasagiline or pharmaceutically acceptable salts thereof and preparation methods therefor (CN101874790A), Rasagiline soft gelatin capsules (EP2285214B1), Rasagiline formulations and processes for their preparation (US7619117B1), Rasagiline formulations (WO2010111264A2).

After oral administration, Rasagiline mesylate is affected by the first-pass effect of the liver, and bioavailability thereof is low, about 36%. Moreover, the main risk source of Rasagiline in use is high blood pressure, which is often called the "cheese effect". (Simpson.G.M. and White K., "Tyramine studies and the safety of MAOI drugs", The Journal of Clinical Psychiatry, 01 Jul 1984, 45(7 Pt 2):59-61). This effect is caused by the inhibition of peripheral MAO, which is found in high concentrations in the stomach. Orally taken Rasagiline is absorbed through the gastrointestinal tract, and patients on a high-tyramine diet are at risk of raising blood pressure at any dose (FDA: AZILECT^{®} (Rasagiline) Tablets: Label). In addition, Parkinson's patients are usually accompanied by gastrointestinal motility disorders, mainly manifested as dysphagia, gastric emptying disorders and constipation (Wolfgang, H.Jost. "Gastrointestinal motility problems in patients with Parkinson's disease" Drugs&Aging, 10, pages 249-258 (1997)), which prolongs the time to inhibit peripheral MAO, and then aggravates the "cheese effect".

Chinese patent application CN200910191252.6 discloses a stable Rasagiline composition, and its Example 11 discloses a film that is attached to the oral mucosa, but the film prepared thereof has poor stability, and the impurity content is as high as 2.6 to 15.33% after stored at 60°C for 10 days.

Further, RAO PINGALI SRINIVASA ET AL: "Formulation, Optimization and Evaluation of Rasagiline Mouth Dissolving Films",INTERNATIONAL JOURNAL OF PHARMACEUTICAL SCIENCES AND NANOTECHNOLOGY, vol. 11, no. 6, 30 November 2018 (2018-11-30), pages 4323-4331, dicloses fast-dissolving oral films (FDOF) of rasagiline; the optimized formulation showed the faster disintegration time i.e., 8±0.18 sec, good percentage elongation (9.4%), good folding endurance (120±4), and the in vitro drug release is 99.39±5.28% within 9 min.

And CN 105 616 389 A discloses an oral fast-dissolving film of loratadine, which has a short melting time, a rapid release of the drug, and does not require water to be swallowed, and has a good mouthfeel.

### CONTENT OF THE PRESENT INVENTION

The technical problem to be solved by the present disclosure is to overcome the defect of poor stability of Rasagiline formulations in the prior art, and to provide a sublingual film of Rasagiline or a pharmaceutically acceptable salt thereof, and a preparation method therefor and a use thereof. In the present disclosure, a drug is made into a sublingual film that is convenient for administration and stable in quality, without the need to be taken with water. The film melts swiftly, and the drug is quickly dissolved from the formulation, and the drug is absorbed through the oral mucosa, which can not only avoid the first-pass effect, but also improve bioavailability; it can also avoid the "cheese effect" caused by the inhibition of peripheral MAO in the stomach and reduce side effects.

Rasagiline is easily oxidized, unstable under acidic and alkaline conditions, and easily soluble in water, with a small dose (less than 1 mg) and a large relative amount of excipients. Therefore, the stability of the drug is greatly affected by the excipients. The inventors found that a variety of excipients are prone to interact with Rasagiline, resulting in a significant increase in the content of oxidized impurities, and the addition of antioxidants still cannot effectively solve the stability problem. In the prescription of the sublingual film prepared by Rasagiline or a pharmaceutical salt thereof, the inventor accidentally discovered through creative labor that a certain amount of one or more of maltodextrin, hydroxypropyl-β-cyclodextrin or glucosyl-β-cyclodextrin which cooperates with other components in the prescription may effectively improve the stability of the film and effectively enhance the stability of the drug during long-term placement.

The present disclosure solves the above technical problem through the following technical solution.

The present disclosure provides a sublingual film of Rasagiline or a pharmaceutically acceptable salt thereof, which comprises the following prescription components:

| | |
|---|---|
| Rasagiline or a pharmaceutical salt | 0.5 to 15% |
| polymer film-forming material | 30 to 85% |
| dextrin | 5% to 40% |
| other excipients | 0 to 30%, but not 0% |

| | |
|---|---|
| the dextrin is one or more of maltodextrin, hydroxypropyl-β-cyclodextrin and glucosyl-β-cyclodextrin; the above percentages are the mass percentages of each component relative to the total components of the prescription of the sublingual film of Rasagiline or the pharmaceutically acceptable salt thereof. | |

In the present disclosure, the Rasagiline or the pharmaceutically acceptable salt thereof is preferably Rasagiline mesylate.

In the present disclosure, the polymer film-forming material can be a conventional water-soluble polymer material that can be used for film formation in the art, such as one or more of hydroxypropyl methylcellulose (HPMC), polyvinyl alcohol (PVA), hydroxypropyl cellulose (HPC), sodium carboxymethylcellulose (CMC-Na), polyvinylpyrrolidone (PVP), polyethylene oxide (PEO), sodium alginate, pullulan, Bletilla gum, corn starch and carrageenan, preferably one or more of hydroxypropyl methylcellulose (HPMC), polyvinyl alcohol (PVA), hydroxypropyl cellulose (HPC), sodium carboxymethylcellulose (CMC-Na), sodium alginate, pullulan and corn starch, such as a mixture of hydroxypropyl methylcellulose (HPMC) and polyvinyl alcohol (PVA), a mixture of hydroxypropyl cellulose (HPC) and sodium carboxymethylcellulose (CMC-Na), a mixture of polyvinyl alcohol (PVA) and corn starch, a mixture of pullulan and polyvinyl alcohol (PVA) or a mixture of hydroxypropyl methylcellulose (HPMC) and sodium alginate.

When the polymer film-forming material is the mixture of hydroxypropyl methylcellulose (HPMC) and polyvinyl alcohol (PVA), the mass ratio of hydroxypropyl methylcellulose (HPMC) to polyvinyl alcohol (PVA) can be (27.5 to 45):(22.5 to 40), such as 45:40, 42.5:37.5, 37.5:32.5, 32.5:27.5 or 27.5:22.5.

When the polymer film-forming material is the mixture of hydroxypropyl cellulose (HPC) and sodium carboxymethylcellulose (CMC-Na), the mass ratio of hydroxypropyl cellulose (HPC) to sodium carboxymethylcellulose (CMC-Na) can be 40:(25 to 35), such as 40:30.

When the polymer film-forming material is the mixture of polyvinyl alcohol (PVA) and corn starch, the mass ratio of polyvinyl alcohol (PVA) to corn starch can be 30:(25 to 35), such as 30:30.

When the polymer film-forming material is the mixture of pullulan and polyvinyl alcohol (PVA) the mass ratio of pullulan to polyvinyl alcohol (PVA) can be 20:(15 to 25), such as 20:20.

When the polymer film-forming material is the mixture of hydroxypropyl methylcellulose (HPMC) and sodium alginate, the mass ratio of hydroxypropyl methylcellulose (HPMC) to sodium alginate can be 20:(5 to 15), such as 20:10.

In the present disclosure, the dextrin is preferably maltodextrin, hydroxypropyl-β-cyclodextrin, glucosyl-β-cyclodextrin, a mixture of maltodextrin and glucosyl-β-cyclodextrin, or a mixture of maltodextrin and hydroxypropyl-β-cyclodextrin.

When the dextrin is the mixture of maltodextrin and glucosyl-β-cyclodextrin, the mass ratio of maltodextrin to glucosyl-β-cyclodextrin is preferably 1:(0.8 to 1.2), such as 1:1.

When the dextrin is the mixture of maltodextrin and hydroxypropyl-β-cyclodextrin, the mass ratio of maltodextrin to hydroxypropyl-β-cyclodextrin is preferably 1:(0.8 to 1.2), such as 1:1.

In the present disclosure, the other excipients can be conventional in the art, preferably comprising one or more of an antioxidant, a disintegrant, a plasticizer, a colorant, an esssence and a sweetener.

Where the disintegrant can be sodium carboxymethyl starch CMS-Na. The amount of the disintegrant in use can be 3 to 7%, such as 5%, and the percentage is the mass percentage relative to the total components of the prescription of the sublingual film of Rasagiline or the pharmaceutically acceptable salt thereof.

Where the plasticizer can be PEG400. The amount of the plasticizer in use can be 7 to 11%, such as 9%, and the percentage is the mass percentage relative to the total components of the prescription of the sublingual film of Rasagiline or the pharmaceutically acceptable salt thereof.

Where the colorant can be titanium dioxide. The amount of the colorant in use can be 2 to 6%, such as 4%, and the percentage is the mass percentage relative to the total components of the prescription of the sublingual film of Rasagiline or the pharmaceutically acceptable salt thereof.

Where the essence can be one or more strawberry essence, grape essence and sweet orange essence. The amount of the essence in use can be 1% or 3%, and the percentage is the mass percentage relative to the total components of the prescription of the sublingual film of Rasagiline or the pharmaceutically acceptable salt thereof.

In the present disclosure, the other excipients can also comprise a sweetener. The sweetener can be conventional in the art, preferably comprising one or more of xylitol, maltitol, steviol glycoside, sodium saccharin, aspartame, acesulfame potassium and sodium cyclamate, more preferably one or more of xylitol, maltitol, aspartame, sodium cyclamate and steviol glycoside, such as xylitol, maltitol, aspartame, sodium cyclamate or steviol glycoside.

Where the amount of the sweetener in use can be conventional in the art, which can be 1%, 4%, 5%, 10% or 30%, preferably 3% to 30%, more preferably 5 to 20%; and the percentage is the mass percentage of the sweetener relative to the total components of the prescription of the sublingual film of Rasagiline or the pharmaceutically acceptable salt thereof.

In the present disclosure, the amount of Rasagiline or the pharmaceutical salt thereof in use can be 1%, 5%, 8%, 10% or 15%, preferably 1 to 8%.

In the present disclosure, the amount of polymer film-forming material in use can be 30%, 40%, 50%, 60%, 70%, 80% or 85%, preferably 40 to 60%. If the amount of the polymer film-forming material is lower than 30%, the film-forming property is not good, and the strength and toughness of the film may not meet the needs of use; if the amount of the polymer film-forming material is higher than 85%, the proportion of other excipients is too small, and the stability, taste and other aspects of the sublingual film of Rasagiline or the pharmaceutically acceptable salt thereof cannot meet the requirements.

In the present disclosure, the amount of dextrin in use can be 5%, 10%, 20%, 25%, 30% or 40%, preferably 10 to 30%. If the amount of dextrin is lower than 5%, the stability of the sublingual film of Rasagiline or the pharmaceutically acceptable salt thereof cannot be significantly improved; if the amount of dextrin is higher than 40%, the film is brittle and easily broken.

In the present disclosure, the amount of the other excipients in use is preferably 2 to 20%, such as 9%, 12%, 15% or 20%.

The present disclosure also provides a preparation method for the sublingual film of Rasagiline or the pharmaceutically acceptable salt thereof, which comprises the following process: coating a slurry to prepare a film;
wherein the slurry comprises the above prescription components of the sublingual film of Rasagiline or the pharmaceutically acceptable salt thereof and water.

In the present disclosure, the slurry can be prepared by conventional methods in the art, preferably by the following method: adding prescription components other than the polymer film-forming material to the aqueous slurry containing the polymer film-forming material, and mixing evenly.

Where, in the aqueous slurry containing the polymer film-forming material, the mass concentration of the polymer film-forming material is preferably 10% to 40%, more preferably 13% to 30%.

In the present disclosure, the amount of water used in the slurry can be conventional in the art, generally based on the amount of water that can be made into a solution with a certain viscosity and good fluidity.

In the present disclosure, the slurry is generally uniform. Before the slurry is coated to prepare the film, the slurry is generally left to stand for defoaming.

In the present disclosure, the method of coating to prepare the film can be conventional in the art, for example, a conventional film coating machine in the art can be used for coating, followed by drying and cutting.

Where, in the coating to prepare the film process, the thickness of the coating film can be 0.3 to 0.5 mm, such as 0.4 mm.

Where the drying temperature can be 80 to 95°C.

Where, in the coating to prepare the film process, the film coating speed can be 40 to 150 cm/min, such as 50 cm/min.

Where the specification of the cut film can be selected according to actual needs.

The present disclosure also provides the sublingual film of Rasagiline or the pharmaceutically acceptable salt thereof for use in the treatment of diseases related to Parkinson's disease or Alzheimer's disease.

The above preferred conditions can be combined arbitrarily to obtain preferred embodiments of the present disclosure by common knowledge in the art.

The reagents and raw materials used in the present disclosure are all commercially available.

The positive and progressive effects of the present disclosure are:
1) The sublingual film of the present disclosure can adhere to the administration site without using water and dissolve quickly. The patient does not need to swallow, and the sublingual film has good compliance and is more convenient for the nursing staff to assist in administration.
2) The drug is absorbed through the oral mucosa, which avoids the first-pass effect of the liver and has high bioavailability, and reduces the dose of the drug compared with oral formulations.
3) The drug dissolves in the mouth and is absorbed through the oral mucosa without going through the gastrointestinal tract, which may avoid the "cheese effect" and reduce adverse responses.
4) The sublingual film of the present disclosure has good stability, and the impurity content is still low after long-term storage.
5) The sublingual film of the present disclosure has good dissolution characteristics, which is characterized by rapid and complete dissolution of the drug, and the drug is quickly absorbed into the blood circulation through the sublingual mucosa, and has a quick effect.

### BRIEF DESCRIPTION OF THE DRAWINGS

FIG. 1 is the dissolution curves of the sublingual films of Prescription 1 in Example 1 and the sublingual films in Examples 2, 3, 5 and Comparative example 4.
FIG. 2 is a curve showing the changes in the plasma concentration of Rasagiline with time after the administration of sublingual film in Example 3, intravenous injection and intragastric administration, respectively.

### DETAILED DESCRIPTION OF THE PREFERRED EMBODIMENT

The present disclosure will be further described below with reference to examples, but the present disclosure is not therefore limited to the scope of the examples. Experimental methods without specific conditions in the following examples are selected according to conventional methods and conditions, or according to the commercial specification.

The raw materials and excipients used in the examples and comparative examples are conventional and commercially available.

The stability investigation, compatibility experiment, and dissolution curve test and the like in the effect examples are all carried out according to the experimental conditions and test methods specified in Pharmacopoeia.

### Example 1

The components and doses of Prescriptions 1 to 5 and Comparative Prescriptions are shown in Table 1.

**Table 1**

| Name of Raw Materials and Excipients | Mass Percentage% | | | | | |
|---|---|---|---|---|---|---|
| | Comparative Prescription | Prescription 1 | Prescription 2 | Prescription 3 | Prescription 4 | Prescription 5 |
| Rasagiline mesylate | 1 | 1 | 1 | 1 | 1 | 1 |
| Hydroxypropyl methylcellulose (HPMC) | 47.5 | 45 | 42.5 | 37.5 | 32.5 | 27.5 |
| Polyvinyl alcohol (PVA) | 42.5 | 40 | 37.5 | 32.5 | 27.5 | 22.5 |
| Hydroxypropyl-β-cyclodextrin | 0 | 5 | 10 | 20 | 30 | 40 |
| Steviol glycoside | 4 | 4 | 4 | 4 | 4 | 4 |
| Titanium dioxide | 4 | 4 | 4 | 4 | 4 | 4 |
| Strawberry essence | 1 | 1 | 1 | 1 | 1 | 1 |
| Total | 100 | 100 | 100 | 100 | 100 | 100 |
| Physical properties of the film | The strength and toughness of the film are good | The strength and toughness of the film are good | The strength and toughness of the film are good | The strength and toughness of the film are good | The strength and toughness of the film can basically meet the needs | The film is slightly brittle, and occasionally there will be edging phenomenon. |

### Preparation processes:

1. Preparation of the aqueous slurry of the film-forming material: According to the ratio of the prescription, HPMC and PVA were dissolved in water to make an aqueous slurry with a mass concentration of 25%.
2. Rasagiline mesylate, hydroxypropyl-β-cyclodextrin, steviol glycoside, titanium dioxide and strawberry essence were added into the above aqueous slurry, and stirred evenly.
3. Standing for defoaming, and the film was coated under the condition that the thickness of the film was 0.4 mm and the drying temperature was 85°C. The speed of coating to prepare the film was 50 cm/min.
4. The mixture was cut and packaged according to the specification of 0.5 mg/tablet.

It can be seen from Table 1 that (1) the physical properties of the films of Prescriptions 1 to 3 of the present disclosure are better in strength and toughness than those of the films of Prescriptions 4 to 5. (2) Although the physical properties of the films of Prescriptions 4 to 5 of the present disclosure are relatively inferior to those of the Comparative Prescriptions, it can be seen from the Effect Example 4 that the Comparative Prescription has poor high-temperature stability and is unqualified. In particular, after being placed at 60°C for 5 days, the content of related substances in the Comparative Prescriptions is as high as 1.33%, which is much higher than that of Prescriptions 1 to 5 of the present disclosure; after being placed at 60°C for 10 days, the content of related substances in the Comparative Prescriptions is as high as 2.35%, which is much higher than that of Prescriptions 1 to 5 of the present disclosure.

### Example 2

The prescription of Example 2 is as shown in Table 2.

**Table 2**

| Name of Raw Materials and Excipients | Mass Percentage% |
|---|---|
| Rasagiline mesylate | 5 |
| Hydroxypropyl cellulose (HPC) | 40 |
| Sodium carboxymethylcellulose (CMC-Na) | 30 |
| Maltodextrin | 5 |
| Glucosyl-β-cyclodextrin | 5 |
| Maltitol | 10 |
| Titanium dioxide | 4 |
| Grape essence | 1 |
| Total | 100 |

### Preparation processes:

1. Preparation of the aqueous slurry of the film-forming material: 40 g of HPC and 30 g of CMC-Na were dissolved in water to make an aqueous slurry with a mass concentration of 20%.
2. Rasagiline mesylate, maltodextrin, glucosyl-β-cyclodextrin, maltitol, titanium dioxide, and grape essence were added into the above aqueous slurry, and stirred evenly.
3. Standing for defoaming, and the film was coated under the condition that the thickness of the film was 0.4 mm and the drying temperature was 85°C. The speed of coating to prepare the film was 50 cm/min.
4. The mixture was cut and packaged according to the specification of 0.5 mg/tablet.

### Example 3

The prescription of Example 3 is as shown in Table 3.

**Table 3**

| Name of Raw Materials and Excipients | Mass Percentage% |
|---|---|
| Rasagiline mesylate | 8 |
| Polyvinyl alcohol PVA | 30 |
| Corn starch | 30 |
| Maltodextrin | 20 |
| Aspartame | 5 |
| Titanium dioxide | 4 |
| Sweet orange essence | 3 |
| Total | 100 |

Preparation processes:
1. Preparation of the aqueous slurry of the film-forming material: 30 g of PVA and 30 g of corn starch were dissolved in water to make an aqueous slurry with a mass concentration of 16%.
2. Rasagiline mesylate, maltodextrin, aspartame, titanium dioxide, and sweet orange essence were added into the above aqueous slurry, and stirred evenly.
3. Standing for defoaming, and the film was coated under the condition that the thickness of the film was 0.4 mm and the drying temperature was 85°C. The speed of coating to prepare the film was 50 cm/min.
4. The mixture was cut and packaged according to the specification of 0.5 mg/tablet.

### Comparative Example 4

The prescription of Comparative Example 4 is as shown in Table 4.

**Table 4**

| Name of Raw Materials and Excipients | Mass Percentage% |
|---|---|
| Rasagiline mesylate | 10 |
| Pullulan | 20 |
| Polyvinyl alcohol (PVA) | 20 |
| Sulfobutyl-β-cyclodextrin | 30 |
| Xylitol | 10 |
| Titanium dioxide | 4 |
| Strawberry essence | 1 |
| Disintegrant: sodium carboxymethyl starch (CMS-Na) | 5 |
| Total | 100 |

### Preparation processes:

1. Preparation of the aqueous slurry of the film-forming material: 20 g of pullulan and 30 g of PVA were dissolved in water to make an aqueous slurry with a mass concentration of 14%.
2. Rasagiline mesylate, sulfobutyl-β-cyclodextrin, xylitol, titanium dioxide, strawberry essence, and CMS-Na were added into the above aqueous slurry, and stirred evenly.
3. Standing for defoaming, and the film was coated under the condition that the thickness of the film was 0.4 mm and the drying temperature was 85°C. The speed of coating to prepare the film was 50 cm/min.
4. The mixture was cut and packaged according to the specification of 0.5 mg/tablet.

### Example 5

The prescription of Example 5 is as shown in Table 5.

**Table 5**

| Name of Raw Materials and Excipients | Mass Percentage% |
|---|---|
| Rasagiline mesylate | 15 |
| Hydroxypropyl methylcellulose (HPMC) | 20 |
| Sodium alginate | 10 |
| Maltodextrin | 20 |
| Hydroxypropyl-β-cyclodextrin | 20 |
| Sodium cyclamate | 1 |
| Titanium dioxide | 4 |
| Strawberry essence | 1 |
| Plasticizer: PEG400 | 9 |
| Total | 100 |

### Preparation processes:

1. Preparation of the aqueous slurry of the film-forming material: 20 g of HPMC and 10 g of sodium alginate were dissolved in water to make an aqueous slurry with a mass concentration of 10%.
2. Rasagiline mesylate, maltodextrin, hydroxypropyl-β-cyclodextrin, sodium cyclamate, titanium dioxide, strawberry essence and PEG400 were added into the above aqueous slurry, and stirred evenly.
3. Standing for defoaming, and the film was coated under the condition that the thickness of the film was 0.4 mm and the drying temperature was 85°C. The speed of coating to prepare the film was 50 cm/min.
4. The mixture was cut and packaged according to the specification of 0.5 mg/tablet.

### Comparative Example 1

The maltodextrin in the prescription of Example 3 was replaced with PVA, and the other processes of the prescription were unchanged.

### Comparative Example 2

The sulfobutyl-β-cyclodextrin in the prescription of Comparative Example 4 was replaced with pullulan, and the other processes of the prescription were unchanged.

### Effect Example 1: Compatibility Test of Raw Materials and Excipients

After the excipients such as polymer film-forming materials and the drug were mixed in a certain proportion, purified water was added to grind evenly. The film was coated, dried, and placed at 60°C for 10 days. The sample was taken on the 5th and 10th days to investigate the variation of related substances. The results are shown in Table 6.

**Table 6 Compatibility Test Results of Raw Materials and Excipients - Polymer Film-Forming Materials**

| Excipients | HPMC | PVA | HPMC + Maltodextrin (10:1) | HPMC + Hydroxypropyl-β-cyclodextrin (10:1) | HPMC + EDTA (10:1) | HPMC + Sodium metabisulfite (10:1) | HPMC + Ascorbic acid (10:1) | HPMC + Sodium bisulfite (10:1) | |
|---|---|---|---|---|---|---|---|---|---|
| Mass ratio of raw materials : | 1:11 | 1:11 | 1:11 | 1:11 | 1:11 | 1:11 | 1:11 | 1:11 | |

| excipients | | | | | | | | | |
|---|---|---|---|---|---|---|---|---|---|
| Total Impurity% | Original | 0.29 | 0.14 | 0.06 | 0.05 | 0.34 | 0.25 | 0.38 | 0.22 |
| | 60°C for 5 days | 1.59 | 0.95 | 0.51 | 0.47 | 1.85 | 1.42 | 1.87 | 1.63 |
| | 60°C for 10 days | 2.54 | 1.86 | 0.76 | 0.65 | 2.97 | 2.39 | 3.51 | 2.12 |

As shown in Table 6, when HPMC and PVA film-forming materials are used alone, or when HPMC is compounded with EDTA, sodium pyrosulfate, ascorbic acid, and sodium bisulfite and many other conventional stabilizers, the total impurity content is much higher than when HPMC is compounded with "maltodextrin or hydroxypropyl-β-cyclodextrin".

Other excipients and the drug were mixed in a certain proportion, followed by being placed at 60°C for 10 days. The sample was taken on the 5th and 10th days to investigate the variation of related substances. The results are shown in Table 7.

**Table 7 Compatibility Test Results of Major Drugs and Excipients - Other Excipients**

| Excipients | | Aspartame | Xylitol | Titanium dioxide | Strawberry essence | PEG400 | CMC-Na |
|---|---|---|---|---|---|---|---|
| Mass ratio of major drugs : excipients | | 1:5 | 1:5 | 1:5 | 1:5 | 1:5 | 1:5 |
| Total Impurity% | Original | 0.28 | 0.95 | 0.26 | 0.08 | 0.14 | 0.22 |
| | 60°C for 5 days | 5.44 | 1.73 | 0.83 | 1.19 | 1.36 | 1.02 |
| | 60°C for 10 days | 11.45 | 2.25 | 1.42 | 3.72 | 2.75 | 1.93 |

The results in Tables 6 to 7 show that the compatibility stability of Rasagiline mesylate and various excipients is poor, and there is still no improvement after adding various conventional stabilizers, but the stability is significantly enhanced after adding dextrin.

### Effect Example 2: Dissolution Curve

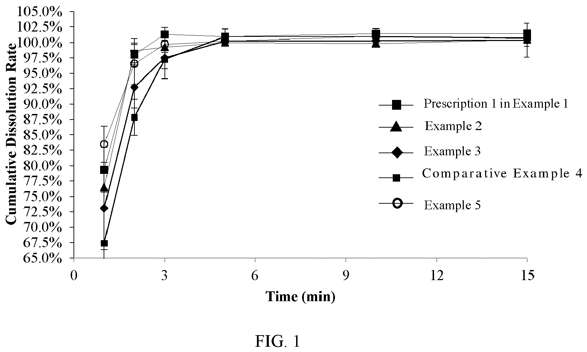

6 films each in Prescription 1 of Example 1 and in Examples 2 to 5 (specification: 0.5 mg, i.e., each film contains 0.5 mg of Rasagiline) were taken, according to the dissolution test method (the second method of the general rule 0931 in the Chinese Pharmacopoeia (2015 edition, volume IV)), with 500 mL of phosphate buffered saline of pH 6.8 as a solvent and 50 rpm of the rotating speed, according to the method. After 1, 2, 3, 5, 10 and 15 min, 1 mL of the each solution was taken to filter, and 20 µL of the continued filtrate was precisely measured and tested according to high-performance liquid chromatography; the dissolution amount at different times was calculated. The dissolution curve is shown in FIG. 1.

The result of FIG. 1 shows that the sublingual film of the Rasagiline mesylate of the present disclosure dissolves rapidly and substantially completely in 3 min.

### Effect Example 3: Pharmacokinetic Test of Beagle Dogs

Intravenous injection: 4 healthy Beagle dogs, half male and half female, were respectively intravenously administered 4 mL of Rasagiline mesylate solution (the solvent was water) (0.5 mg/mL, calculated as Rasagiline), i.e., the administration dose was 2 mg.

Sublingual film and intragastric administration: A two-period double crossover test was adopted, and the washout period was 1 week. 4 healthy Beagle dogs, half male and half female, were randomly divided into two groups, respectively sublingually administered the sublingual film of Rasagiline mesylate (Example 3) and intragastrically administered Rasagiline mesylate solution, and the administration dose was 2 mg.

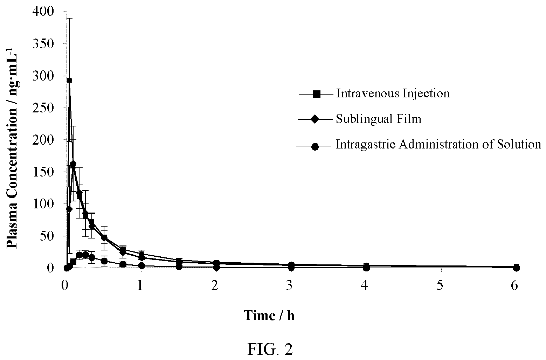

The dogs were fasted for 16 hours before administration and had free access to water. After 4 hours of administration, the dogs had free access to water, and food was administered. 2 mL of blood was collected from the forelimb or hindlimb vein before administration (0 h) and 0.033 h, 0.083 h, 0.167 h, 0.25 h, 0.333 h, 0.5 h, 0.75 h, 1 h, 1.5 h, 2 h, 3 h, 4 h, 6 h after administration, and placed in heparin sodium anticoagulation tubes. The collected blood samples were centrifuged immediately (1006×g, 10 min), separated to obtain the plasma and stored at -20°C. Using Selegiline hydrochloride as the internal standard and processing by using acetonitrile protein precipitation method, the plasma concentration of Rasagiline in the sample was determined by LC-MS/MS, and the average drug-time curve was drawn (FIG. 2). The non-compartmental model of DAS2.0 software was used to process the plasma concentration data and calculate the bioavailability of Rasagiline.

The result in FIG. 2 shows that after intragastric administration of Rasagiline mesylate to Beagle dogs, the bioavailability is low due to the first-pass effect, and the absolute bioavailability is 11.8%±3.3% (n=4). After sublingual administration of sublingual film of Rasagiline mesylate in the present disclosure, the drug is absorbed through the sublingual mucosa, and the bioavailability is significantly increased, and the absolute bioavailability is 77.8%±9.7% (n=4); increased to 6.9±1.3 times that of intragastric administration (n=4); and the onset of effect is rapid, Tmax is (0.07±0.02) h (n=4).

### Effect Example 4: Stability Investigation of Samples Containing Different Proportions of Dextrin in Prescriptions

The films of Comparative Prescription and Prescriptions 1 to 5 in Example 1 (specification: 0.5 mg) were taken, and the outer packaging was removed, and a high temperature test was conducted to investigate the stability of the sample.

The film was placed in a culture dish at 60°C for 10 days, and samples were taken on the 5th and 10th days to test the related substances of Rasagiline in the film. The test results show that the proportion of hydroxypropyl-β-cyclodextrin is in the range of 5 to 40%, and the stability of the drug under high temperature environment is enhanced with the increase of the amount of hydroxypropyl-β-cyclodextrin. However, with the increase of the amount of hydroxypropyl-β-cyclodextrin, the amount of film-forming materials needs to be reduced accordingly. The strength of the film gradually decreases, and the toughness gradually deteriorates. The results are shown in Table 8.

**Table 8 Stability Investigation Result in Example 1**

| Investigation Item | | Original | 60°C for 5 days | 60°C for 10 days |
|---|---|---|---|---|
| Related Substance (%) | Comparative Prescription | 0.13 | 1.33 | 2.35 |
| | Prescription 1 | 0.10 | 0.57 | 0.79 |
| | Prescription 2 | 0.09 | 0.49 | 0.63 |
| | Prescription 3 | 0.08 | 0.31 | 0.46 |
| | Prescription 4 | 0.08 | 0.25 | 0.39 |
| | Prescription 5 | 0.06 | 0.21 | 0.35 |

### Effect Example 5

### Stability Investigation of Different Prescription Samples

The films in Comparative Example 1, Comparative Example 2, Comparative Example 4 and Examples 2, 3 and 5 (specification: 0.5 mg) were taken, and the outer packaging was removed, and a high temperature test was conducted to investigate the stability of the sample.

The film was placed in a culture dish at 60°C for 10 days, and samples were taken on the 5th and 10th days to test the content, related substances and dissolution rate of Rasagiline in the film (see Effect Example 2 for the dissolution method). The test result shows that during the investigation period, the related substances of the sublingual film of Rasagiline mesylate produced in Comparative Example 1 and Comparative Example 2 increase significantly, and the results are shown in Tables 9 to 10. The sublingual films of Rasagiline mesylate prepared in the present disclosure, i.e, Examples 2, 3 and 5; and Comparative Example 4, have no obvious change in each index and have stable product quality, and the results are shown in Tables 11 to 14.

Table 9 Stability Investigation Results in Comparative Example 1

| Investigation Item | Original | 60°C for 5 days | 60°C for 10 days |
|---|---|---|---|
| Related substance (%) | 0.10 | 1.27 | 1.96 |
| Dissolution Rate in 3 Minutes (%) | 98.7 | 97.5 | 96.2 |
| Content (%) | 100.2 | 98.9 | 97.4 |

**Table 10 Stability Investigation Results in Comparative Example 2**

| Investigation Item | Original | 60°C for 5 days | 60°C for 10 days |
|---|---|---|---|
| Related Substance (%) | 0.14 | 1.46 | 2.59 |
| Dissolution Rate in 3 Minutes (%) | 99.6 | 97.5 | 96.1 |
| Content (%) | 101.3 | 99.2 | 98.1 |

**Table 11 Stability Investigation Results in Example 2**

| Investigation Item | Original | 60°C for 5 days | 60°C for 10 days |
|---|---|---|---|
| Related Substance (%) | 0.07 | 0.41 | 0.64 |
| Dissolution Rate in 3 Minutes (%) | 99.6 | 98.9 | 98.1 |
| Content (%) | 101.0 | 100.5 | 100.6 |

**Table 12 Stability Investigation Results in Example 3**

| Investigation Item | Original | 60°C for 5 days | 60°C for 10 days |
|---|---|---|---|
| Related Substance (%) | 0.09 | 0.37 | 0.52 |
| Dissolution Rate in 3 Minutes (%) | 99.6 | 98.8 | 99.4 |
| Content (%) | 101.8 | 100.5 | 101.4 |

**Table 13 Stability Investigation Results in Comparative Example 4**

| Investigation Item | Original | 60°C for 5 days | 60°C for 10 days |
|---|---|---|---|
| Related Substance (%) | 0.05 | 0.33 | 0.41 |
| Dissolution Rate in 3 Minutes (%) | 99.8 | 98.5 | 99.3 |
| Content (%) | 100.5 | 101.2 | 100.4 |

**Table 14 Stability Investigation Results in Example 5:**

| Investigation Item | Original | 60°C for 5 days | 60°C for 10 days |
|---|---|---|---|
| Related Substance (%) | 0.08 | 0.36 | 0.49 |
| Dissolution Rate in 3 Minutes (%) | 98.9 | 97.4 | 98.3 |
| Content (%) | 101.3 | 100.0 | 101.2 |

The results in Tables 9 to 14 show that due to the synergistic effect of each component, the total impurity of the sublingual film of Rasagiline mesylate of the present disclosure does not exceed 1% after storing at a high temperature of 60°C for 10 days, and the stability increases with the increase of the proportion of dextrin. Compared with Comparative Example 1 and Comparative Example 2 without dextrin added, after being placed at high temperature, there is no obvious variation in the dissolution rate and content, and the increase of related substances is obviously reduced, and the stability is obviously enhanced.

In the present disclosure, the oral film of Rasagiline does not need water or other liquids when taken. The packaging bag is torn open with dry hands, and the film is taken out, and placed in the oral cavity until completely dissolved. This product should not be folded, chewed or swallowed. Feeding or drinking should be avoided within 10 min after dosing.

### Effect Example 6

### Stability Investigation of Samples with Different Water Content

### Stability Investigation of Samples with Different Water Content

Films with different water content were prepared according to the prescription and preparation method of Example 3 (column 2 in the table below), packaged and sealed in PET/Al/PE bags, and placed at 60°C for 10 days. The samples were taken on the 5th and 10th days to test the related substances in the film. The test results are shown in Table 15.

**Table 15 Stability Investigation Results of Different Water Content**

| NO. | Water Content (%) | Related Substance (%) | | |
|---|---|---|---|---|
| | | 0 d | 60°C for 5 days | 60°C for 10 days |
| 1 | 2.2 | 0.10 | 0.52 | 0.67 |
| 2 | 4.7 | 0.09 | 0.40 | 0.49 |
| 3 | 6.0 | 0.08 | 0.30 | 0.42 |
| 4 | 7.7 | 0.09 | 0.25 | 0.35 |
| 5 | 10.8 | 0.10 | 0.18 | 0.26 |
| 6 | 13.2 | 0.09 | 0.15 | 0.22 |

The results in Table 15 show that water content has an effect on the stability of the sublingual film of Rasagiline mesylate of the present disclosure, and as the water content increases, the stability of the sublingual film of the present disclosure is enhanced.

Although the specific implementations of the present disclosure have been described above, those skilled in the art should understand that these are only examples, and various changes or modifications can be made to these implementations without departing from the principle and essence of the present disclosure. Accordingly, the protection scope of the present disclosure is defined by the appended claims.

## Claims

1. A sublingual film of Rasagiline or a pharmaceutically acceptable salt thereof, wherein the sublingual film comprises the following prescription components:
| | |
|---|---|
| Rasagiline or a pharmaceutical salt | 0.5 to 15% |
| polymer film-forming material | 30 to 85% |
| dextrin | 5% to 40% |
| other excipients | 0 to 30%, and not 0% |
the dextrin is one or more of maltodextrin, hydroxypropyl-β-cyclodextrin and glucosyl-β-cyclodextrin;
the above percentages are the mass percentages of each component relative to the total components of the prescription of the sublingual film of Rasagiline or the pharmaceutically acceptable salt thereof.

2. The sublingual film of Rasagiline or the pharmaceutically acceptable salt thereof as claimed in claim 1, wherein the Rasagiline or the pharmaceutically acceptable salt thereof is Rasagiline mesylate;
and/or, the polymer film-forming material is one or more of hydroxypropyl methylcellulose HPMC, polyvinyl alcohol PVA, hydroxypropyl cellulose HPC, sodium carboxymethylcellulose CMC-Na, polyvinylpyrrolidone PVP, polyethylene oxide PEO, sodium alginate, pullulan, Bletilla gum, corn starch and carrageenan,
and/or, the dextrin is maltodextrin, hydroxypropyl-β-cyclodextrin, glucosyl-β-cyclodextrin, a mixture of maltodextrin and glucosyl-β-cyclodextrin, or a mixture of maltodextrin and hydroxypropyl-β-cyclodextrin;
and/or, the other excipients comprise one or more of an antioxidant, a disintegrant, a plasticizer, a colorant, an essence and a sweetener.

3. The sublingual film of Rasagiline or the pharmaceutically acceptable salt thereof as claimed in claim 2, wherein the polymer film-forming material is one or more of hydroxypropyl methylcellulose HPMC, polyvinyl alcohol PVA, hydroxypropyl cellulose HPC, sodium carboxymethylcellulose CMC-Na, sodium alginate, pullulan and corn starch, preferably a mixture of hydroxypropyl methylcellulose HPMC and polyvinyl alcohol PVA, a mixture of hydroxypropyl cellulose HPC and sodium carboxymethylcellulose CMC-Na, a mixture of polyvinyl alcohol PVA and corn starch, a mixture of pullulan and polyvinyl alcohol PVA, or a mixture of hydroxypropyl methylcellulose HPMC and sodium alginate.

4. The sublingual film of Rasagiline or the pharmaceutically acceptable salt thereof as claimed in claim 2 or 3, wherein when the polymer film-forming material is the mixture of hydroxypropyl methylcellulose HPMC and polyvinyl alcohol PVA, the mass ratio of hydroxypropyl methylcellulose HPMC to polyvinyl alcohol PVA is (27.5 to 45):(22.5 to 40), such as 45:40, 42.5:37.5, 37.5:32.5, 32.5:27.5 or 27.5:22.5;
when the polymer film-forming material is the mixture of hydroxypropyl cellulose HPC and sodium carboxymethylcellulose CMC-Na, the mass ratio of hydroxypropyl cellulose HPC to sodium carboxymethylcellulose CMC-Na is 40:(25 to 35), such as 40:30;
when the polymer film-forming material is the mixture of polyvinyl alcohol PVA and corn starch, the mass ratio of polyvinyl alcohol PVA to corn starch is 30:(25 to 35), such as 30:30;
when the polymer film-forming material is the mixture of pullulan and polyvinyl alcohol PVA, the mass ratio of pullulan to polyvinyl alcohol PVA is 20:(15 to 25), such as 20:20;
when the polymer film-forming material is the mixture of hydroxypropyl methylcellulose HPMC and sodium alginate, the mass ratio of hydroxypropyl methylcellulose HPMC to sodium alginate is 20:(5 to 15), such as 20:10.

5. The sublingual film of Rasagiline or the pharmaceutically acceptable salt thereof as claimed in at least one of claims 2 to 4, wherein, when the dextrin is the mixture of maltodextrin and glucosyl-β-cyclodextrin, the mass ratio of maltodextrin to glucosyl-β-cyclodextrin is 1 :(0.8 to 1.2), such as 1:1;
when the dextrin is the mixture of maltodextrin and hydroxypropyl-β-cyclodextrin, the mass ratio of maltodextrin to hydroxypropyl-β-cyclodextrin is 1:(0.8 to 1.2), such as 1:1.

6. The sublingual film of Rasagiline or the pharmaceutically acceptable salt thereof as claimed in at least one of claims 2 to 5, wherein the disintegrant is sodium carboxymethyl starch CMS-Na; the amount of the disintegrant in use is preferably 3 to 7%, such as 5%, and the percentage is the mass percentage relative to the total components of the prescription of the sublingual film of Rasagiline or the pharmaceutically acceptable salt thereof;
and/or, the plasticizer is PEG400; the amount of the plasticizer in use is preferably 7 to 11%, such as 9%, and the percentage is the mass percentage relative to the total components of the prescription of the sublingual film of Rasagiline or the pharmaceutically acceptable salt thereof;
and/or, the colorant is titanium dioxide; the amount of the colorant in use is preferably 2 to 6%, such as 4%, and the percentage is the mass percentage relative to the total components of the prescription of the sublingual film of Rasagiline or the pharmaceutically acceptable salt thereof;
and/or, the essence is one or more of strawberry essence, grape essence and sweet orange essence; the amount of the essence in use is preferably 1 to 3%, and the percentage is the mass percentage relative to the total components of the prescription of the sublingual film of Rasagiline or the pharmaceutically acceptable salt thereof;
and/or, the sweetener comprises one or more of xylitol, maltitol, steviol glycoside, sodium saccharin, aspartame, acesulfame potassium and sodium cyclamate, preferably one or more of xylitol, maltitol, aspartame, sodium cyclamate and steviol glycoside, such as xylitol, maltitol, aspartame, sodium cyclamate or steviol glycoside;
wherein the amount of the sweetener in use is, for example, 1%, 4%, 5%, 10% or 30%, preferably 3% to 30%, more preferably 5 to 20%; and the above percentage is the mass percentage of the sweetener relative to the total components of the prescription of the sublingual film of Rasagiline or the pharmaceutically acceptable salt thereof.

7. The sublingual film of Rasagiline or the pharmaceutically acceptable salt thereof as claimed in at least one of claims 1 to 6, wherein, the amount of Rasagiline or the pharmaceutical salt in use is 1%, 5%, 8%, 10% or 15%, preferably 1 to 8%;
and/or, the amount of the polymer film-forming material in use is 30%, 40%, 50%, 60%, 70%, 80% or 85%, preferably 40 to 60%;
and/or, the amount of the dextrin in use is 5%, 10%, 20%, 25%, 30% or 40%, preferably 10 to 30%;
and/or, the amount of the other excipients in use is 2 to 20%, such as 9%, 12%, 15% or 20%.

8. A preparation method for the sublingual film of Rasagiline or the pharmaceutically acceptable salt thereof as claimed in at least one of claims 1 to 7, wherein the preparation method comprises the following process: coating a slurry to prepare a film;
wherein the slurry comprises prescription components of the sublingual film of Rasagiline or the pharmaceutically acceptable salt thereof, and water.

9. The preparation method for the sublingual film of Rasagiline or the pharmaceutically acceptable salt thereof as claimed in claim 8, wherein the slurry is prepared by the following method: adding prescription components other than the polymer film-forming material to an aqueous slurry containing the polymer film-forming material, and mixing evenly;
wherein, in the aqueous slurry containing the polymer film-forming material, the mass concentration of the polymer film-forming material is preferably 10% to 40%, more preferably 13% to 30%;
in the coating to prepare the film process, the thickness of the coating film is preferably 0.3 to 0.5 mm, such as 0.4 mm;
in the coating to prepare the film process, the film coating speed is preferably 40 to 150 cm/min, such as 50 cm/min.

10. The sublingual film of Rasagiline or the pharmaceutically acceptable salt thereof as claimed in at least one of claims 1 to 7 for use in the treatment of diseases related to Parkinson's disease or Alzheimer's disease.

## Patentansprüche

1. Sublingualer Film aus Rasagilin oder einem pharmazeutisch verträglichen Salz davon, wobei der sublinguale Film die folgenden Rezepturbestandteile umfasst:
Rasagilin oder ein pharmazeutisches Salz 0,5 bis 15 %
polymerfilmbildendes Material 30 bis 85 %
Dextrin 5 % bis 40 %
andere Arzneimittelträgersubstanzen 0 bis 30 %, und nicht 0 %
das Dextrin eines oder mehrere aus Maltodextrin, Hydroxypropyl-P-Cyclodextrin und Glucosyl-P-Cyclodextrin ist;
die oben genannten Prozentsätze die Massenanteile der einzelnen Bestandteile relativ zu den Gesamtbestandteilen der Rezeptur des sublingualen Films aus Rasagilin oder dem pharmazeutisch verträglichen Salz davon sind.

2. Sublingualer Film aus Rasagilin oder dem pharmazeutisch verträglichen Salz davon nach Anspruch 1, wobei das Rasagilin oder das pharmazeutisch verträgliche Salz davon Rasagilinmesilat ist;
und/oder das polymerfilmbildende Material eines oder mehrere aus Hydroxypropylmethylcellulose, HPMC, Polyvinylalkohol, PVA, Hydroxypropylcellulose, HPC, Natriumcarboxymethylcellulose, CMC-Na, Polyvinylpyrrolidon, PVP, Polyethylenoxid, PEO, Natriumalginat, Pullulan, Bletillagummi, Maisstärke und Carrageenan ist;
und/oder das Dextrin Maltodextrin, Hydroxypropyl-β-Cyclodextrin, Glucosyl-β-Cyclodextrin, eine Mischung aus Maltodextrin und Glucosyl-β-Cyclodextrin oder eine Mischung aus Maltodextrin und Hydroxypropyl-β-Cyclodextrin ist;
und/oder die anderen Arzneimittelträgersubstanzen eines oder mehrere von einem Antioxidationsmittel, einem Sprengmittel, einem Weichmacher, einem Farbstoff, einer Essenz und einem Süßstoff umfassen.

3. Sublingualer Film aus Rasagilin oder dem pharmazeutisch verträglichen Salz davon nach Anspruch 2, wobei das polymerfilmbildende Material eines oder mehrere aus Hydroxypropylmethylcellulose HPMC, Polyvinylalkohol PVA, Hydroxypropylcellulose HPC, Natriumcarboxymethylcellulose CMC-Na, Natriumalginat, Pullulan und Maisstärke, bevorzugt eine Mischung aus Hydroxypropylmethylcellulose HPMC und Polyvinylalkohol PVA, eine Mischung aus Hydroxypropylcellulose HPC und Natriumcarboxymethylcellulose CMC-Na, eine Mischung aus Polyvinylalkohol PVA und Maisstärke, eine Mischung aus Pullulan und Polyvinylalkohol PVA oder eine Mischung aus Hydroxypropylmethylcellulose HPMC und Natriumalginat ist.

4. Sublingualer Film aus Rasagilin oder dem pharmazeutisch verträglichen Salz davon nach Anspruch 2 oder 3, wobei, wenn das polymerfilmbildende Material die Mischung aus Hydroxypropylmethylcellulose, HPMC, und Polyvinylalkohol, PVA ist, das Massenverhältnis von Hydroxypropylmethylcellulose, HPMC, zu Polyvinylalkohol, PVA (27,5 bis 45):(22,5 bis 40), wie beispielsweise 45:40, 42,5:37,5, 37,5:32,5, 32,5:27,5 oder 27,5:22,5 beträgt;
wenn das polymerfilmbildende Material die Mischung aus Hydroxypropylcellulose, HPC, und Natriumcarboxymethylcellulose, CMC-Na ist, das Massenverhältnis von Hydroxypropylcellulose, HPC, zu Natriumcarboxymethylcellulose, CMC-Na 40:(25 bis 35), wie beispielsweise 40:30 beträgt;
wenn das polymerfilmbildende Material die Mischung aus Polyvinylalkohol, PVA, und Maisstärke ist, das Massenverhältnis von Polyvinylalkohol, PVA, zu Maisstärke 30:(25 bis 35), wie beispielsweise 30:30 beträgt;
wenn das polymerfilmbildende Material die Mischung aus Pullulan und Polyvinylalkohol, PVA, ist, das Massenverhältnis von Pullulan zu Polyvinylalkohol, PVA, 20:(15 bis 25), wie beispielsweise 20:20 beträgt;
wenn das polymerfilmbildende Material die Mischung aus Hydroxypropylmethylcellulose, HPMC, und Natriumalginat ist, das Massenverhältnis von Hydroxypropylmethylcellulose, HPMC, zu Natriumalginat 20:(5 bis 15), wie beispielsweise 20:10 beträgt.

5. Sublingualer Film aus Rasagilin oder dem pharmazeutisch verträglichen Salz davon nach zumindest einem der Ansprüche 2 bis 4, wobei, wenn das Dextrin die Mischung aus Maltodextrin und Glucosyl-β-Cyclodextrin ist, das Massenverhältnis von Maltodextrin zu Glucosyl-β-Cyclodextrin 1:(0,8 bis 1,2), wie beispielsweise 1:1 beträgt;
wenn das Dextrin die Mischung aus Maltodextrin und Hydroxypropyl-β-Cyclodextrin ist, das Massenverhältnis von Maltodextrin zu Hydroxypropyl-β-Cyclodextrin 1:(0,8 bis 1,2), wie beispielsweise 1:1 beträgt.

6. Sublingualer Film aus Rasagilin oder dem pharmazeutisch verträglichen Salz davon nach zumindest einem der Ansprüche 2 bis 5, wobei das Sprengmittel Natriumcarboxymethylstärke, CMS-Na, ist; die verwendete Menge des Sprengmittels bevorzugt 3 bis 7 %, wie beispielsweise 5 % beträgt, und der Prozentsatz der Massenanteil relativ zu den Gesamtbestandteilen der Rezeptur des sublingualen Films aus Rasagilin oder dem pharmazeutisch verträglichen Salz davon ist;
und/oder der Weichmacher PEG400 ist; die verwendete Menge des Weichmachers bevorzugt 7 bis 11 %, wie beispielsweise 9 % beträgt, und der Prozentsatz der Massenanteil relativ zu den Gesamtbestandteilen der Rezeptur des sublingualen Films aus Rasagilin oder dem pharmazeutisch verträglichen Salz davon ist;
und/oder der Farbstoff Titandioxid ist; die verwendete Menge des Farbstoffs bevorzugt 2 bis 6 %, wie beispielsweise 4 % beträgt, und der Prozentsatz der Massenanteil relativ zu den Gesamtbestandteilen der Rezeptur des sublingualen Films aus Rasagilin oder dem pharmazeutisch verträglichen Salz davon ist;
und/oder die Essenz eine oder mehrere aus Erdbeeressenz, Traubenessenz und Süßorangenessenz ist; die verwendete Menge der Essenz bevorzugt 1 bis 3 % beträgt, und der Prozentsatz der Massenanteil relativ zu den Gesamtbestandteilen der Rezeptur des sublingualen Films aus Rasagilin oder dem pharmazeutisch verträglichen Salz davon ist;
und/oder der Süßstoff eines oder mehrere aus Xylit, Maltit, Steviol-Glycosid, Kristallose, Aspartam, Acesulfam und Natriumzyklamat, bevorzugt eines oder mehrere aus Xylit, Maltit, Aspartam, Natriumzyklamat und Steviol-Glycosid, wie beispielsweise Xylit, Maltit, Aspartam, Natriumzyklamat oder Steviol-Glycosid umfasst;
wobei die verwendete Menge des Süßstoffs zum Beispiel 1 %, 4 %, 5 %, 10 % oder 30 %, bevorzugte 3 % bis 30 %, bevorzugter 5 bis 20 % beträgt; und der oben genannte Prozentsatz der Massenanteil des Süßstoffs relativ zu den Gesamtbestandteilen der Rezeptur des sublingualen Films aus Rasagilin oder dem pharmazeutisch verträglichen Salz davon ist.

7. Sublingualer Film aus Rasagilin oder dem pharmazeutisch verträglichen Salz davon nach zumindest einem der Ansprüche 1 bis 6, wobei die verwendete Menge von Rasagilin oder dem pharmazeutisch verträglichen Salz 1 %, 5 %, 8 %, 10 % oder 15 %, bevorzugt 1 bis 8 % beträgt;
und/oder die verwendete Menge des polymerfilmbildenden Materials 30 %, 40 %, 50 %, 60 %, 70 %, 80 % oder 85 %, bevorzugt 40 bis 60 % beträgt;
und/oder die verwendete Menge des Dextrins 5 %, 10 %, 20 %, 25 %, 30 % oder 40 %, bevorzugt 10 bis 30 % beträgt;
und/oder die verwendete Menge der anderen Arzneimittelträgersubstanzen 2 bis 20 %, wie beispielsweise 9 %, 12 %, 15 % oder 20 % beträgt.

8. Herstellungsverfahren für den sublingualen Film aus Rasagilin oder dem pharmazeutisch verträglichen Salz davon nach zumindest einem der Ansprüche 1 bis 7, wobei das Herstellungsverfahren den folgenden Prozess umfasst: Beschichten einer Suspension zum Herstellen eines Films;
wobei die Suspension Rezepturbestandteil des sublingualen Films aus Rasagilin oder des pharmazeutisch verträglichen Salzes davon und Wasser umfasst.

9. Herstellungsverfahren für den sublingualen Film aus Rasagilin oder dem pharmazeutisch verträglichen Salz davon nach Anspruch 8, wobei die Suspension durch das folgende Verfahren hergestellt wird: Hinzufügen von Rezepturbestandteilen außer dem polymerfilmbildenden Material zu einer wässrigen Suspension, welche das polymerfilmbildende Material enthält, und gleichmäßiges Mischen;
wobei in der wässrigen Suspension, welche das polymerfilmbildende Material enthält, die Massenkonzentration des polymerfilmbildenden Materials bevorzugt 10 % bis 40 %, bevorzugter 13 % bis 30 % beträgt;
in dem Beschichtungsprozess zum Herstellen des Films die Dicke des Beschichtungsfilms bevorzugt 0,3 bis 0,5 mm, beispielsweise 0,4 mm beträgt;
in dem Beschichtungsprozess zum Herstellen des Films die Filmbeschichtungsgeschwindigkeit bevorzugt 40 bis 150 cm/min, wie beispielsweise 50 cm/min beträgt.

10. Sublingualer Film aus Rasagilin oder dem pharmazeutisch verträglichen Salz davon nach zumindest einem der Ansprüche 1 bis 7 zur Verwendung bei der Behandlung von Erkrankungen, welche mit der Parkinson-Krankheit oder der Alzheimer-Krankheit in Zusammenhang stehen.

## Revendications

1. Film sublingual de rasagiline ou du sel pharmaceutiquement acceptable de celle-ci, dans lequel le film sublingual comprend les composants de prescription suivants :
rasagiline ou un sel pharmaceutique 0,5 à 15 %
matériau de formation de film polymère 30 à 85 %
dextrine 5 % à 40 %
autres excipients 0 à 30 %, et pas 0 %
la dextrine est une ou plusieurs parmi la maltodextrine, la hydroxypropyl-P-cyclodextrine et la glucosyl-P-cyclodextrine ;
les pourcentages ci-dessus sont les pourcentages massiques de chaque composant par rapport aux composants totaux de la prescription du film sublingual de rasagiline ou du sel pharmaceutiquement acceptable de celle-ci.

2. Film sublingual de rasagiline ou du sel pharmaceutiquement acceptable de celle-ci selon la revendication 1, dans lequel la rasagiline ou le sel pharmaceutiquement acceptable de celle-ci est le mésylate de rasagiline ;
et/ou le matériau de formation de film polymère est un ou plusieurs parmi l'hydroxypropylméthylcellulose, HPMC, l'alcool polyvinylique, PVA, l'hydroxypropylcellulose, HPC, la carboxyméthylcellulose sodique, CMC-Na, la polyvinylpyrrolidone, PVP, l'oxyde de polyéthylène, PEG, l'alginate de sodium, le pullulane, la gomme Bletilla, l'amidon de maïs et la carraghénine,
et/ou la dextrine est la maltodextrine, l'hydroxypropyl-β-cyclodextrine, la glucosyl-β-cyclodextrine, un mélange de maltodextrine et de glucosyl-β-cyclodextrine, ou un mélange de maltodextrine et d'hydroxypropyl-β-cyclodextrine ;
et/ou les autres excipients comprennent un ou plusieurs parmi un antioxydant, un désintégrant, un plastifiant, un colorant, une essence et un édulcorant.

3. Film sublingual de rasagiline ou du sel pharmaceutiquement acceptable de celle-ci selon la revendication 2, dans lequel le matériau de formation de film polymère est un ou plusieurs parmi l'hydroxypropylméthylcellulose, HPMC, l'alcool polyvinylique, PVA, l'hydroxypropylcellulose, HPC, la carboxyméthylcellulose sodique, CMC-Na, l'alginate de sodium, le pullulane et l'amidon de maïs, de préférence un mélange d'hydroxypropylméthylcellulose, HPMC, et d'alcool polyvinylique, PVA, un mélange d'hydroxypropylcellulose, HPC, et de carboxyméthylcellulose sodique, CMC-Na, un mélange d'alcool polyvinylique, PVA, et d'amidon de maïs, un mélange de pullulane et d'alcool polyvinylique, PVA, ou un mélange d'hydroxypropylméthylcellulose, HPMC, et d'alginate de sodium.

4. Film sublingual de rasagiline ou du sel pharmaceutiquement acceptable de celle-ci selon la revendication 2 ou 3, dans lequel, lorsque le matériau de formation de film polymère est le mélange d'hydroxypropylméthylcellulose, HPMC, et d'alcool polyvinylique, PVA, le rapport massique entre l'hydroxypropylméthylcellulose, HPMC, et l'alcool polyvinylique, PVA, est (27,5 à 45):(22,5 à 40), comme 45:40, 42,5:37,5, 37,5:32,5, 32,5:27,5 ou 27,5:22,5 ;
lorsque le matériau de formation de film polymère est le mélange d'hydroxypropylcellulose, HPC, et de carboxyméthylcellulose sodique, CMC-Na, le rapport massique entre l'hydroxypropylcellulose, HPC, et la carboxyméthylcellulose sodique, CMC-Na, est 40:(25 à 35), comme 40:30 ;
lorsque le matériau de formation de film polymère est le mélange d'alcool polyvinylique, PVA, et d'amidon de maïs, le rapport massique entre l'alcool polyvinylique, PVA, et l'amidon de maïs est 30:(25 à 35), comme 30:30 ;
lorsque le matériau de formation de film polymère est le mélange de pullulane et d'alcool polyvinylique, PVA, le rapport massique entre le pullulane et l'alcool polyvinylique, PVA, est 20:(15 à 25), comme 20:20 ;
lorsque le matériau de formation de film polymère est le mélange d'hydroxypropylméthylcellulose, HPMC, et d'alginate de sodium, le rapport massique entre l'hydroxypropylméthylcellulose, HPMC, et l'alginate de sodium est 20:(5 à 15), comme 20:10.

5. Film sublingual de rasagiline ou du sel pharmaceutiquement acceptable de celle-ci selon au moins l'une des revendications 2 à 4, dans lequel, lorsque la dextrine est le mélange de maltodextrine et de glucosyl-β-cyclodextrine, le rapport massique entre la maltodextrine et la glucosyl-β-cyclodextrine est 1:(0,8 à 1,2), comme 1:1 ;
lorsque la dextrine est le mélange de maltodextrine et d'hydroxypropyl-β-cyclodextrine, le rapport massique entre la maltodextrine et l'hydroxypropyl-β-cyclodextrine est 1:(0,8 à 1,2), comme 1:1.

6. Film sublingual de rasagiline ou du sel pharmaceutiquement acceptable de celle-ci selon au moins l'une des revendications 2 à 5, dans lequel le désintégrant est le carboxyméthylamidon sodique, CMS-Na ; la quantité du désintégrant lors de l'utilisation est, de préférence, 3 à 7 %, comme 5 %, et le pourcentage est le pourcentage massique par rapport aux composants totaux de la prescription du film sublingual de rasagiline ou du sel pharmaceutiquement acceptable de celle-ci ;
et/ou le plastifiant est le PEG400 ; la quantité du plastifiant lors de l'utilisation est, de préférence, 7 à 11 %, comme 9 %, et le pourcentage est le pourcentage massique par rapport aux composants totaux de la prescription du film sublingual de rasagiline ou du sel pharmaceutiquement acceptable de celle-ci ;
et/ou le colorant est le dioxyde de titane ; la quantité du colorant lors de l'utilisation est, de préférence, 2 à 6 %, comme 4 %, et le pourcentage est le pourcentage massique par rapport aux composants totaux de la prescription du film sublingual de rasagiline ou du sel pharmaceutiquement acceptable de celle-ci ;
et/ou l'essence est une ou plusieurs parmi l'essence de fraise, l'essence de raisin et l'essence d'orange douce ; la quantité de l'essence lors de l'utilisation est, de préférence, 1 à 3 % et le pourcentage est le pourcentage massique par rapport aux composants totaux de la prescription du film sublingual de rasagiline ou du sel pharmaceutiquement acceptable de celle-ci ;
et/ou l'édulcorant comprend un ou plusieurs parmi le xylitol, le maltitol, le glycoside de stéviol, la saccharine sodique, l'aspartame, l'acésulfame de potassium et le cyclamate de sodium, de préférence un ou plusieurs parmi le xylitol, le maltitol, l'aspartame, le cyclamate de sodium et le glycoside de stéviol, comme le xylitol, le maltitol, l'aspartame, le cyclamate de sodium ou le glycoside de stéviol ;
dans lequel la quantité de l'édulcorant lors de l'utilisation est, par exemple, 1%, 4 %, 5 %, 10 % ou 30 %, de préférence 3 % à 30 %, plus préférentiellement 5 à 20 % ; et le pourcentage ci-dessus est le pourcentage massique de l'édulcorant par rapport aux composants totaux de la prescription du film sublingual de rasagiline ou du sel pharmaceutiquement acceptable de celle-ci.

7. Film sublingual de rasagiline ou du sel pharmaceutiquement acceptable de celle-ci selon au moins l'une des revendications 1 à 6, dans lequel la quantité de rasagiline ou du sel pharmaceutique lors de l'utilisation est 1 %, 5 %, 8 %, 10 % ou 15 %, de préférence 1 à 8%;
et/ou la quantité du matériau de formation de film polymère lors de l'utilisation est 30 %, 40 %, 50 %, 60 %, 70 %, 80 % ou 85 %, de préférence 40 à 60 % ;
et/ou la quantité de la dextrine lors de l'utilisation est 5 %, 10 %, 20 %, 25 %, 30 % ou 40 %, de préférence 10 à 30 % ;
et/ou la quantité des autres excipients lors de l'utilisation est 2 à 20 %, comme 9 %, 12 %, 15 % ou 20 %.

8. Procédé de préparation du film sublingual de rasagiline ou du sel pharmaceutiquement acceptable de celle-ci selon au moins l'une des revendications 1 à 7, dans lequel le procédé de préparation comprend le processus suivant : l'enrobage d'une suspension pour préparer un film ;
dans lequel la suspension comprend des composants de prescription du film sublingual de rasagiline ou du sel pharmaceutiquement acceptable de celle-ci, et de l'eau.

9. Procédé de préparation du film sublingual de rasagiline ou du sel pharmaceutiquement acceptable de celle-ci selon la revendication 8, dans lequel la suspension est préparée par le procédé suivant : l'ajout de composants de prescription autres que le matériau de formation de film polymère à une suspension aqueuse contenant le matériau de formation de film polymère, et le mélange homogène ;
dans lequel, dans la suspension aqueuse contenant le matériau de formation de film polymère, la concentration massique du matériau de formation de film polymère est, de préférence, 10 % à 40 %, plus préférentiellement 13 % à 30 % ;
dans le processus d'enrobage pour préparer le film, l'épaisseur du film d'enrobage est, de préférence, 0,3 à 0,5 mm, comme 0,4 mm ;
dans le processus d'enrobage pour préparer le film, la vitesse d'enrobage du film est, de préférence, 40 à 150 cm/min, comme 50 cm/min.

10. Film sublingual de rasagiline ou du sel pharmaceutiquement acceptable de celle-ci selon au moins l'une des revendications 1 à 7, destiné à être utilisé dans le traitement de maladies liées à la maladie de Parkinson ou à la maladie d'Alzheimer.
